# EUROPÄISCHE PATENTSCHRIFT

(11) **EP 1 741 397 B1**
(45) Veröffentlichungstag und Bekanntmachung des Hinweises auf die Patenterteilung: **14.07.2010**
(21) Anmeldenummer: 06019616.9
(22) Anmeldetag: 26.08.2003
(51) Int. Cl.: A61B 17/80

(54) **Knochenplatte**
Bone plate
Plaque d'ostéosynthèse

(43) Veröffentlichungstag der Anmeldung: 10.01.2007
(62) Teilanmeldung aus: 03818256.4
(73) Patentinhaber: Synthes GmbH, 4436 Oberdorf (CH)
(72) Erfinder: Schneider, Rolf, 2562 Port (CH)
(74) Vertreter: Rosenich, Paul

(56) Entgegenhaltungen:
- EP-A- 0 530 585
- WO-A-2005/018472
- WO-A-2007/014279
- DE-A- 4 343 117

## Beschreibung

Die Erfindung bezieht sich auf eine Knochenplatte gemäß dem Oberbegriff des Patentanspruchs 1.

Solche Knochenplatten eigenen sich für Indikationen am gesamten Skelett. Von besonderer Bedeutung sind aber die üblichen Groß- und Kleinfragment-Indikationen für die operative Knochenbruchbehandlung.

Aus der DE-A 198 32 513 ist eine gattungsgemäße Knochenplatte bekannt. Die Abwinkelung der Knochenschrauben relativ zur Knochenplatte und ihre winkelstabile Fixierung wird bei dieser bekannten Vorrichtung durch einen zwischen dem Schraubenkopf und dem Plattenloch angeordneten Ring bewirkt. Nachteilig bei dieser Konstruktion ist einerseits die aufwändigere Herstellung mit einem zusätzlichen Bauteil (Ring), die Gefahr, dass der winzige Ring aus dem Plattenloch fällt oder aus diesem herausgedrückt wird, was die Vorrichtung unbrauchbar macht, anderseits die aufwändigere OP-Technik, da der Ring vor dem Einbringen der Schraube entsprechend der Achse ausgerichtet werden muss.

Die DE 43 43 117 A1 zeigt eine Knochenplatte mit einer knochenseitigen Unterseite, einer Oberseite und mehreren die Unterseite mit der Oberseite verbindenden Plattenlöchern, die jeweils eine zentrale Lochachse, eine sich gegen die Unterseite hin verjüngende Innenmantelfläche und einen Gewindegang aufweisen. Die Innenmantelflächen weisen jeweils 3 oder 4 sich radial von der Lochachse weg erstreckende Ausnehmungen auf, welche den Gewindegang unterbrechen.

Hier will die Erfindung Abhilfe schaffen. Der Erfindung liegt die Aufgabe zugrunde, eine Knochenplatte zu schaffen, welche in der Lage ist - ohne zusätzliche Bauelemente - herkömmliche Kopfverriegelungsschrauben winkel- und achsstabil aufzunehmen.

Die Erfindung löst die gestellte Aufgabe mit einer Knochenplatte, welche die Merkmale des Anspruchs 1 aufweist.

Der durch die Erfindung erreichte Vorteil ist im wesentlichen darin zu sehen, dass dank der erfindungsgemäßen Knochenplatte eine Kopfverriegelungsschraube als Knochenschraube auch in einem von der vorgegebenen (üblicherweise orthogonal auf der Plattenebene stehenden) Lochachse abweichenden Winkel eingebracht und in dieser Position verriegelt werden kann, ohne dadurch - wie bei bekannten Vorrichtungen - signifikant an Stabilität einzubüßen.

Durch die mindestens drei Ausnehmungen in der Innenmantelfläche des Plattenlochs entstehen - auch bei einer Schräglage der Kopfverriegelungsschraube - zentrierende Auflager für den Schraubenkopf, welche zu einer gleichmäßigen Lastverteilung führen. Bei Kopfverriegelungsschrauben mit einem Kopfgewinde und Plattenlöcher mit einem Innengewinde kann das Kopfgewinde - bei einer Schräglage der Schraube - die durch die Ausnehmungen unterbrochenen Gewindegänge im Plattenloch "überspringen", ohne sie jedoch zu "überschneiden".

Ein weiterer Vorteil der erfindungsgemäßen Knochenplatte besteht in der Möglichkeit die mindestens drei Ausnehmungen im Plattenloch zur Führung von Bohr- oder Führungsbüchsen zu verwenden, mit welchen die Kopfverriegelungsschrauben beim Einbringen geführt werden können. Die Bohr- oder Führungsbüchsen brauchen dabei nicht mehr - wie beim Stand der Technik - in die Plattenlöcher eingeschraubt zu werden, sondern können, dank der Ausnehmungen, lediglich in die Plattenlöcher eingesteckt werden, was auf eine einfache Art Zentrum und Richtung der Lochachse ergibt. Die kannulierten Bohr- oder Führungsbüchsen brauchen dazu an ihrer Spitze lediglich die Negativform der Plattenloch-Geometrie aufzuweisen, ohne irgendwelche Gewinde oder sonstige, gleichwirkende Strukturen. Gegebenenfalls kann auch ein Schnappmechanismus damit verbunden werden.

Weitere vorteilhafte Ausgestaltungen der Erfindung sind in den abhängigen Ansprüchen gekennzeichnet.

Bei einer besonderen Ausführungsform ist die Innenmantelfläche des Plattenlochs mit einer dreidimensionalen Strukturierung versehen. Diese dient der Führung einer entsprechend strukturierten Kopfverriegelungsschraube. Die dreidimensionale Strukturierung ist makroskopisch und besteht vorzugsweise aus partiellen oder vollständigen Gewindegängen, Rippen oder Erhebungen. Die Innenmantelfläche kann auch ein mehrgängiges Gewinde tragen.

Die Oberflächengeometrie der durch die N Ausnehmungen gebildeten N "Lochschenkel" ist dabei vorteilhafterweise so zu gestalten, dass die Kompatibilität mit der einzubringenden Kopfverriegelungsschraube begünstigt wird. Dies kann in Form eines klassischen helixförmigen Gewindes, eine gewindeähnliche Form mit oder ohne Steigung oder auch nur eine bestimmte Anzahl von Nuten oder Rippen sein, oder auch ein Quasi-Gewinde mit oder ohne Steigung geschehen. Die Anzahl der Nuten oder Rippen ist vorzugsweise immer ungerade (z. B. 3,5, 7 oder 9).

Die Innenmantelfläche kann konkav, vorzugsweise sphärisch, konisch oder ellipsoid ausgebildet sein. Diese Form begünstigt den Einlauf einer Kopfverriegelungsschraube derart, dass beim ersten Kontakt der Kopfverriegelungsschraube mit der Innenmantelfläche, die Kopfverriegelungsschraube automatisch ins Plattenloch hineingezogen wird, ohne vorher über die Knochenplatte eine Kompressionskraft auf den Knochen auszuüben, wie dies bei Vorrichtungen gemäß dem Stand der Technik teilweise der Fall ist.

Bei einer weiteren Ausführungsform ist mindesten eines der Plattenlöcher als Langloch ausgebildet.

Die N Ausnehmungen sind jeweils in einem Abstand von 360°/N von der Zentralachse aus gesehen angeordnet. Die Ausnehmungen weisen vorteilhafterweise eine periphere Ausdehnung von mindestens1° und höchstens 119° auf. Die N Ausnehmungen unterteilen dabei die Innenmantelfläche in N Mantelflächenabschnitte.

Bei einer besonderen Ausführungsform verlaufen die Ausnehmungen ausschließlich im Bereich der Innenmantelfläche. Bei einer anderen Ausführungsform erstrecken sich die Ausnehmungen radial von der Lochachse weg über den Bereich der Innenmantelfläche hinaus.

Die Ausnehmungen können sich von der Oberseite zur Unterseite hin zylindrisch oder konisch erstrecken. Dadurch ergibt sich der Vorteil, dass die Ausnehmungen zur Fixierung einer Bohrbüchse zum Vorbohren oder zum Einbringen von Kirschner- Drähten verwendet werden können. Die Bohrbüchse braucht somit nicht mehr in das Plattenloch eingeschraubt, sondern nur noch eingesteckt zu werden, ohne dabei die Schraubenauflager zu verletzen.

Die Ausnehmungen können sich über die gesamte Höhe der Knochenplatte von der Oberseite bis zur Unterseite hin erstrecken.

Die Knochenplatte kann aus Stahl oder Titan oder auch aus einem Kunststoff bestehen. Bei Kunststoffplatten werden solche aus Polyacryletherketon (PEAK) oder Polyetheretherketon (PEEK) mit einer Bruchdehnung von 40-70% und einem Elastizitätsmodul von 3000-6000N/mm² bevorzugt. Es kann aber Polysulfon mit einer Bruchdehnung von 80-120% und einem Elastizitätsmodul von 2000-3500N/mm² verwendet werden. Weiter eignet sich auch Liquid-Cristal-Polymer (LCP) mit einer Bruchdehnung von 1,5-2,5% und einem Elastizitätsmodul von 5000-20'000N/mm². Schließlich können auch Polyoxymethylen (POM) mit einer Bruchdehnung von 10-50% und einem Elastizitätsmodul von 2000-3'500N/mm² und Polyphenylensulfid (PPS) mit einer Bruchdehnung von 0,2-1,0% und einem Elastizitätsmodul von 12000- 20'000N/mm² verwendet werden Die Knochenplatten aus Kunststoff können auch mit Metall-, Kunststoff- oder Kohlenstoff-Fasern verstärkt sein.

Mit den Knochenplatten können verschiedenartige Kopfverriegelungsschrauben verwendet werden. Beispielsweise solche mit einem konvexen, vorzugsweise sphärischen oder konischen Kopfteil. Der Kopfteil der Kopfverriegelungsschrauben kann auch eine dreidimensionale Strukturierung aufweisen. Bei einer speziellen Ausführungsform ist der Kopfteil der Kopfverriegelungsschraube aus einem härteren Material gefertigt als die Innenmantelfläche der Knochenplatte. Vorzugsweise weist die Innenmantelfläche der Knochenplatte und der Kopfteil der Kopfverriegelungsschraube aufeinander abgestimmte Gewinde auf.

Bei einer Kunststoffplatte können die Plattenlöcher auch als metallische Gewindeeinsätze ausgebildet sein. Umgekehrt können bei einer metallischen Knochenplatte die Plattenlöcher als polymere Gewindeeinsatz ausgebildet sind.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 einen Längsschnitt durch eine Knochenplatte mit konischen Plattenlöchern;
Fig. 2 einen Längsschnitt durch eine Knochenplatte mit sphärischen Plattenlöchem;
Fig. 3 eine Aufsicht auf eine Knochenplatte mit 3 Ausnehmungen in der Innenmantelfläche der Plattenlöcher;
Fig. 4 eine Variante der Knochenplatte nach Fig. 3 mit stärkeren Ausnehmungen in der Innenmantelfläche der Plattenlöcher;
Fig. 5 eine Aufsicht auf eine Knochenplatte mit Gewindeeinsätzen mit 4 Ausnehmungen in der Innenmantelfläche elliptisch ausgebildeter Plattenlöcher;
Fig. 6 eine perspektivische Ansicht einer Knochenplatte nach Fig. 1 von oben mit eingesetzten Kopfverriegelungsschrauben;
Fig. 7 eine perspektivische Ansicht einer Knochenplatte nach Fig. 1 von unten mit eingesetzten Kopfverriegelungsschrauben;
Fig. 8 einen Längsschnitt durch eine Knochenplatte mit darin eingesetzter Kopfverriegelungsschraube ohne Abwinkelung ; und
Fig. 9 einen Längsschnitt durch eine Knochenplatte mit darin eingesetzter Kopfverriegelungsschraube mit Abwinkelung.

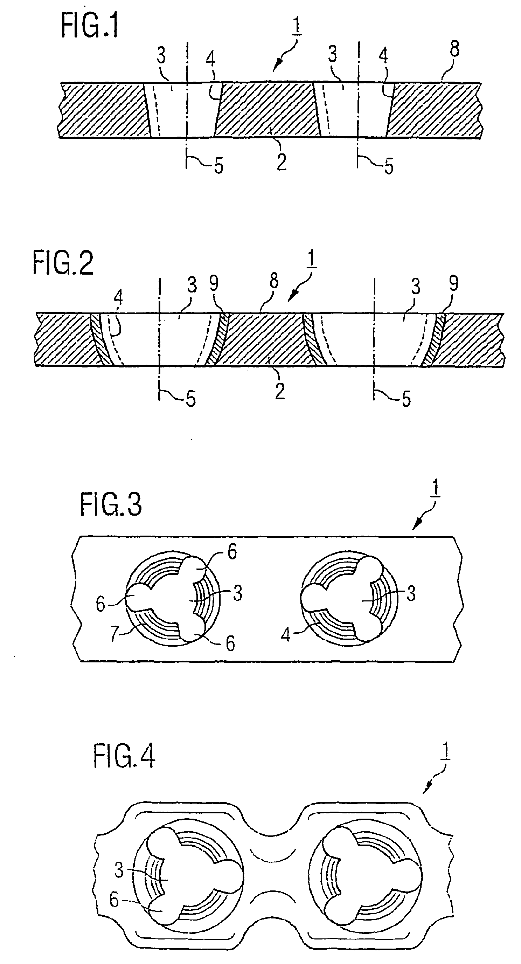

Die in den Fig. 1 und 3 dargestellte Knochenplatte 1 besitzt eine knochenseitige Unterseite 2, eine Oberseite 8 und mehrere die Unterseite 2 mit der Oberseite 8 verbindende Plattenlöcher 3 mit einer zentralen Lochachse 5. Die Plattenlöcher 3 weisen eine sich gegen die Unterseite 2 hin konisch verjüngende Innenmantelfläche 4 auf. Im Weiteren weist die Innenmantelfläche 4 drei sich radial von der Lochachse 5 weg erstreckende Ausnehmungen 6 in einem regelmäßigen Abstand von120° auf. Ihre periphere Ausdehnung beträgt ca.40° und sie verlaufen ausschließlich im Bereich der Innenmantelfläche 4. Die Ausnehmungen 6 erstrecken sich über die gesamte Höhe der Knochenplatte 1 von der Oberseite 8 bis zur Unterseite 2 hin konisch. Die Innenmantelfläche 4 ist zudem mit einer dreidimensionalen Strukturierung 7 in Form eines Gewindes versehen.

In Fig. 4 ist eine Variante der Ausführung nach Fig. 3 dargestellt, bei welcher sich die Ausnehmungen radial von der Lochachse weg über den Bereich der Innenmantelfläche hinaus erstrecken.

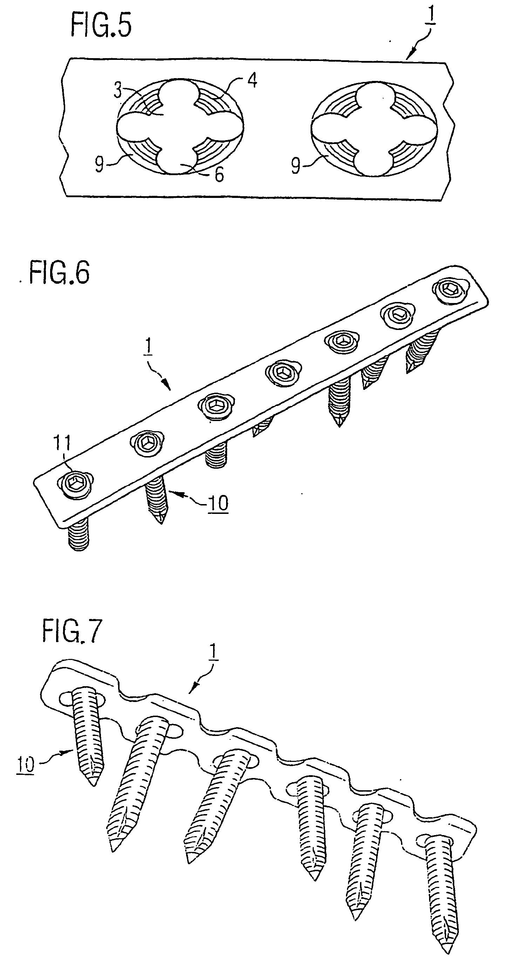

In den Fig. 2 und 5 ist eine weitere alternative Ausführungsform dargestellt, bei welcher die Plattenlöcher 3 als Langlöcher ausgebildet sind. Die Knochenplatte besteht zur Hauptsache aus einem Kunststoff (PEEK) mit darin eingelegten metallischen Gewindeeinsätzen 9 aus Titan, welche die Plattenlöcher 3 bilden. Die Plattenlöcher 3 weisen bei dieser Ausführungsform vier Ausnehmungen 6 auf, die sich radial von der Lochachse 5 weg über den Bereich der Innenmantelfläche 4 hinaus erstrecken. Die Innenmantelfläche 4 wird in vier Mantelflächenabschnitte unterteilt. Die Ausnehmungen erstrecken sich über die gesamte Höhe der Knochenplatte 1 von der Oberseite 8 bis zur Unterseite 2 hin konisch. Die Innenmantelfläche 4 ist zudem mit einer dreidimensionalen Strukturierung 7 in Form eines mehrgängigen Gewindes versehen.

Diese Ausführungsform kann materialmäßig auch invertiert werden, indem die Knochenplatte zur Hauptsache aus Metall (Titan) besteht mit darin eingelegten Gewindeeinsätzen 9 aus Kunststoff (PEEK), welche die Plattenlöcher 3 bilden.

In Fig. 6 ist die Knochenplatte nach Fig. 1 mit von oben eingesetzten Kopfverriegelungsschrauben 10 dargestellt, deren Kopfteil 11 sphärisch ausgebildet ist.

Fig. 7 zeigt die gleiche Knochenplatte 1 von unten.

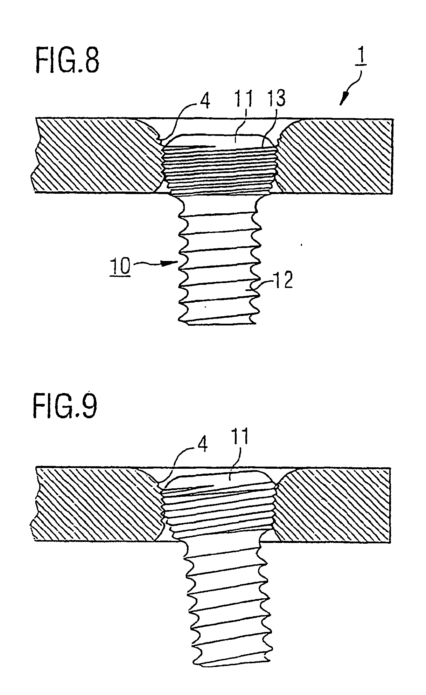

In Fig. 8 ist eine Knochenplatte 1 mit darin eingesetzter Kopfverriegelungsschraube als Knochenschraube 10 ohne Abwinkelung dargestellt. Die Innenmantelfläche 4 des Plattenlochs der Knochenplatte 1 und der Kopfteil 11 der Kopfverriegelungsschraube 10 weise aufeinander abgestimmte Gewinde 13 auf.

In Fig. 9 ist die gleich Variante wie in Fig. 8 dargestellt, wobei jedoch die Kopfverriegelungsschraube 10 abgewinkelt ist.

Die folgenden Ausführungsformen sind möglich, unter anderen. Sie müssen aber auch die Merkmale des Anspruchs 1 aufweisen, um erfindungsgemäß zu sein:
1. Knochenplatte (1) mit einer knochenseitigen Unterseite (2), einer Oberseite (8) und mehreren die Unterseite (2) mit der Oberseite (8) verbindenden Plattenlöcher (3) mit einer zentralen Lochachse (5) und wovon mindestens eines dieser Plattenlöcher (3) eine sich gegen die Unterseite (2) hin verjüngende Innenmantelfläche (4) aufweist, dadurch gekennzeichnet, dass die Innenmantelfläche (4) N≥3 sich radial von der Lochachse (5) weg erstreckende Ausnehmungen (6) aufweist.
2. Knochenplatte (1) nach Ausführungsform 1, dadurch gekennzeichnet, dass die Innenmantelfläche (4) mit einer dreidimensionalen Strukturierung (7) versehen ist.
3. Knochenplatte (1) nach Ausführungsform 1 oder 2, dadurch gekennzeichnet, dass die Innenmantelfläche (4) konkav, vorzugsweise sphärisch, konisch oder ellipsoid ausgebildet ist.
4. Knochenplatte (1) nach einer der Ausführungsformen 1 bis 3, dadurch gekennzeichnet, dass mindesten eines der Plattenlöcher (3) als Langloch ausgebildet ist.
5. Knochenplatte (1) nach einer der Ausführungsformen 1 bis 4, dadurch gekennzeichnet, dass die dreidimensionale Strukturierung makroskopisch ist und vorzugsweise aus partiellen oder vollständigen Gewindegängen, Rippen oder Erhebungen besteht.
6. Knochenplatte (1) nach einer der Ausführungsformen 1 bis 5, dadurch gekennzeichnet, dass die N Ausnehmungen (6) jeweils in einem Abstand von 360°/N von der Zentralachse (5) aus gesehen angeordnet sind.
7. Knochenplatte (1) nach einer der Ausführungsformen 1 bis 6, dadurch gekennzeichnet, dass die Ausnehmungen (6) eine periphere Ausdehnung von mindestens1° aufweisen.
8. Knochenplatte (1) nach einer der Ausführungsformen 1 bis 7, dadurch gekennzeichnet, dass die Ausnehmungen (6) eine periphere Ausdehnung von höchstens 119° aufweisen.
9. Knochenplatte (1) nach einer der Ausführungsformen 1 bis 8, dadurch gekennzeichnet, dass die Ausnehmungen (6) ausschließlich im Bereich der Innenmantelfläche (4) verlaufen.
10. Knochenplatte (1) nach einer der Ausführungsformen 1 bis 9, dadurch gekennzeichnet, dass die Ausnehmungen (6) sich radial von der Lochachse (5) weg über den Bereich der Innenmantelfläche (4) hinaus erstrecken.
11. Knochenplatte (1) nach einer der Ausführungsformen 1 bis 10, dadurch gekennzeichnet, dass die N Ausnehmungen (6) die Innenmantelfläche (4) in N Mantelflächenabschnitte unterteilt.
12. Knochenplatte (1) nach einer der Ausführungsformen 1 bis 11, dadurch gekennzeichnet, sich die Ausnehmungen (6) von der Oberseite (8) zur Unterseite (2) hin zylindrisch oder konisch erstrecken.
13. Knochenplatte (1) nach einer der Ausführungsformen 1 bis 12, dadurch gekennzeichnet, dass sich die Ausnehmungen (6) über die gesamte Höhe der Knochenplatte (1) von der Oberseite (8) bis zur Unterseite (2) hin erstrecken.
14. Knochenplatte (1) nach einer der Ausführungsformen 1-13, dadurch gekennzeichnet, dass sie aus Stahl oder Titan besteht.
15. Knochenplatte (1) nach einer der Ausführungsformen 5-14, dadurch gekennzeichnet, dass die Innenmantelfläche (4) ein mehrgängiges Gewinde trägt.
16. Knochenplatte (1) nach einer der Ausführungsformen 5 bis 15, dadurch gekennzeichnet, dass die Innenmantelfläche (4) quer zur Lochachse (5) peripher umlaufenden Rippen aufweist.
17. Knochenplatte (1) nach einer der Ausführungsformen 1 bis 16, dadurch gekennzeichnet, dass die Knochenplatte (1) aus einem Kunststoff gefertigt ist.
18. Knochenplatte (1) nach Ausführungsform 17, dadurch gekennzeichnet, dass sie aus Polyacryletherketon (PEAK) oder Polyetheretherketon (PEEK) mit einer Bruchdehnung von 40-70% und einem Elastizitätsmodul von 3000-6000N/mm² gefertigt ist.
19. Knochenplatte (1) nach Ausführungsform 17, dadurch gekennzeichnet, dass sie aus Polysulfon mit einer Bruchdehnung von 80-120% und einem Elastizitätsmodul von 2000-3500 N/mm² gefertigt ist.
20. Knochenplatte (1) nach Ausführungsform 17, dadurch gekennzeichnet, dass sie aus Liquid- Cristal-Polymer (LCP) mit einer Bruchdehnung von 1,5-2, 5 % und einem Elastizitätsmodul von 5000-20'000 N/mm² gefertigt ist.
21. Knochenplatte (1) nach Ausführungsform 17, dadurch gekennzeichnet, dass sie aus Polyoxymethylen (POM) mit einer Bruchdehnung von 10-50% und einem Elastizitätsmodul von 2000-3'500 N/mm² gefertigt ist.
22. Knochenplatte (1) nach Ausführungsform 17, dadurch gekennzeichnet, dass sie aus Polyphenylensulfid (PPS) mit einer Bruchdehnung von 0,2-1,0% und einem Elastizitätsmodul von 12000-20'000 N/mm² gefertigt ist.

## Patentansprüche

1. Knochenplatte (1) mit einer knochenseitigen Unterseite (2), einer Oberseite (8) und mehreren die Unterseite (2) mit der Oberseite (8) verbindenden Plattenlöchern (3), die jeweils eine zentrale Lochachse (5), eine sich gegen die Unterseite (2) hin verengend Innenmantelfläche und einen Gewindegang aufweisen, wobei
die Innenmantelflächen (4) jeweils N ≥ 3 sich radial von der Lochachse (5) weg erstreckende Ausnehmungen (6) aufweisen, welche den Gewindegang unterbrechen, **dadurch gekennzeichnet, dass**
die Gewindegänge in den Plattenlöchern (3) und die sie unterbrechenden Ausnehmungen jeweils derart ausgebindet sind, dass das Kopfgewinde einer eingesetzten Kopfverriegelungsschraube bei einer Schräglage dieser Kopfverriegelungsschraube die durch die Ausnehmungen unterbrochenen Gewindegänge im Plattenloch überspringt, ohne sie dabei zu überschneiden.

2. Knochenplatte (1) nach Anspruch 1, **dadurch gekennzeichnet, dass** die Innenmantelfläche (4) mit einer dreidimensionalcn Strukturierung (7) versehen ist.

3. Knochenplatte (1) nach Anspruch 1 oder 2, **dadurch gekennzeichnet, dass** die Innenmantelfläche (4) konkav, vorzugsweise sphärisch, konisch oder ellipsoid ausgebildet ist.

4. Knochenplatte (1) nach einem der Ansprüche 1 bis 3, **dadurch gekennzeichnet, dass** die N Ausnehmungen (6) jeweils in einem Abstand von 360°/N von der Zentralachse (5) aus gesehen angeordnet sind.

5. Knochenplatte (1) nach einem der Ansprüche 1 bis 4, **dadurch gekennzeichnet, dass** die Ausnehmungen (6) ausschließlich im Bereich, der Innenmantelfläche (4) verlaufen.

6. Knochenplatte (1) nach einem der Ansprüche 1 bis 5, **dadurch gekennzeichnet, dass** die Ausnehmungen (6) sich radial von der Lochachse (5) weg über den Bereich der Innenmantelfläche (4) hinaus erstrecken.

7. Knochenplatte (1) nach einem der Ansprüche 1 bis 6, **dadurch gekennzeichnet**, sich die Ausnehmungen (6) von der Oberseite (8) zur Unterseite (2) hin zylindrisch oder konisch erstrecken.

8. Knochenptatte (1) nach einem der Ansprüche 1 bis 7, **dadurch gekennzeichnet, dass** sich die Ausnehmungen (6) über die gesamte Höhe der Knochenplatte (1) von der Oberseite (8) bis zur Unterseite (2) hin erstrecken.

9. Knochenplatte (I) nach einem der Absprüche 1 bis 8, **dadurch gekennzeichnet, dass** sie mindestens eine Kopfverriegelungschraube als Knochenschraube (10) umfasst mit einem zur winkelstabilen Verankerung in das mindestens eine Plattenloch (3) geeigneten sich verjüngenden Kopfteil (11) und einem zur Verankerung im Knochen bestimmten Gewindeteil (12).

10. Knochenplatte (1) nach Anspruch 9, **dadurch gekennzeichnet, dass** der Kopfteil (11) konvex, vorzugweise sphärisch oder konisch ausgebildet ist.

11. Knochenplatte (1) nach Anspruch 9 oder 10, **dadurch gekennzeichnet, dass** die Inneamantelfläche (4) und der Kopfteil (5) der Kopfvorrigelungsschraube (10) aufeinander abgestimmte Gewinde aufweisen.

12. Knochenplatte (1) nach einem der vorhergehenden Ansprüche, **dadurch gekennzeichnet, dass** die periphere Ausdehnung der Ausnehmungen jeweils etwa 40° beträgt.

## Claims

1. Bone plate (1) with an underside (2) on the bone side, an upper side (8) and several plate holes (3) connecting the underside (2) with the upper side (8), which respectively have a central hole axis (5), an inner surface area narrowing towards the underside (2) and a thread pitch, wherein
the inner surface areas (4) have respectively N ≥3 recesses (6) extending radially away from the hole axis (5), which interrupt the thread pitch, **characterized in that**
the thread pitches are formed in the plate holes (3) and the recesses interrupting them respectively such that the head thread of an inserted head locking screw, with an oblique position of this head locking screw, skips over the thread pitches in the plate hole, interrupted by the recesses, without thereby overlapping them.

2. Bone plate (1) according to Claim 1, **characterized in that** the inner surface area (4) is provided with a three-dimensional structuring (7).

3. Bone plate (1) according to Claim 1 or 2, **characterized in that** the inner surface area (4) is constructed so as to be concave, preferably spherical, conical or ellipsoid.

4. Bone plate (1) according to one of Claims 1 to 3, **characterized in that** the N recesses (6) are respectively arranged at a distance or 360°/N viewed from the central axis (5).

5. Bone plate (1) according to one of Claims 1 to 4, **characterized in that** the recesses (6) run exclusively in the region of the inner surface area (4).

6. Bone plate (1) according to one of Claims 1 to 5, **characterized in that** the recesses (6) extend radially away from the hole axis (5) beyond the region of the inner surface area (4).

7. Bone plate (1) according to one of Claims 1 to 6, **characterized in that** the recesses (6) extend cylindrically or conically from the upper side (8) towards the underside (2).

8. Bone plate (1) according to one of Claims 1 to 7, **characterized in that** the recesses (6) extend over the entire height of the bone plate (1) from the upper side (8) up to the underside (2).

9. Bone plate (1) according to one of Claims 1 to 8, **characterized in that** it comprises at least one head flocking screw as bone screw (10) with a narrowing head part (11), suitable for angle-stable anchoring into the at least one plate hole (3), and a thread part (12) intended for anchoring in the bone.

10. Bone plate (1) according to Claim 9, **characterized in that** the head part (11) is constructed so as to be convex, preferably spherical or conical.

11. Bone plate (1) according to Claim 9 or 10, **characterized in that** the inner surface area (4) and the head part (5) of the head locking screw (10) have threads which are coordinated with each other.

12. Bone plate (1) according to one of the preceding claims, **characterized in that** the peripheral extent of the recesses is respectively approximately 40°.

## Revendications

1. Plaque osseuse (1) comportant une face inférieure placée côté os (2), une face supérieure (8) et plusieurs trous dans la plaque (3) reliant la face inférieure (2) à la face supérieure (8) et qui présentent respectivement un axe de perforation central (5), une surface d'enveloppe interne se rétrécissant en direction de la face inférieure (2) et un pas de vis,
les surface d'enveloppe interne (4) présentant des évidements (6) s'étendant radialement depuis l'axe de perforation (5) et interrompant le pas de vis, **caractérisée en ce que**
les pas de vis des trous de la plaque (3) est les évidements les interrompant étant réalisés respectivement de manière à ce que le filet de tête d'une vis de verrouillage de tête insérée, en cas de position oblique de cette vis de verrouillage de tête, saute les pas de vis interrompus par les évidements dans le trou de la plaque sans les croiser.

2. Plaque osseuse (1) selon la revendication 1, **caractérisée en ce que** la surface d'enveloppe interne (4) est pourvue d'une structuration tridimensionnelle (7).

3. Plaque osseuse (1) selon la revendication 1 ou 2, **caractérisée en ce que** la surface d'enveloppe interne (4) est de forme concave, de préférence sphérique, conique ou ellipsoïde.

4. Plaque osseuse (1) selon une des revendications 1 à 3, **caractérisée en ce que** les N évidements (6) sont disposés respectivement avec un écart de 360°/N, vu depuis l'axe central (5).

5. Plaque osseuse (1) selon une des revendications 1 à 4, **caractérisée en ce que** les évidements (6) s'étendent exclusivement au niveau de la surface d'enveloppe interne (4).

6. Plaque osseuse (1) selon une des revendications 1 à 5, **caractérisée en ce que** les évidements (6) s'étendent radialement depuis l'axe de perforation (5) au-delà du niveau de la surface d'enveloppe interne (4).

7. Plaque osseuse (1) selon une des revendications 1 à 6, **caractérisée en ce que** les évidements (6) s'étendent en forme cylindrique ou conique de la face supérieure (8) à la face inférieure (2).

8. Plaque osseuse (1) selon une des revendications 1 à 7, **caractérisée en ce que** les évidements (6) s'étendent sur toute la hauteur de la plaque osseuse (1) de la face supérieure (8) vers la face intérieure (2).

9. Plaque osseuse (1) selon une des revendications 1 à 8, **caractérisée en ce qu'**elle comporte au moins une vis de verrouillage de tête servant de vis à os (10) avec une pièce filetée (12) destinée à un ancrage angulaire stable dans l'au moins une pièce de tête adaptée (11) se rétrécissant dans la plaque perforée (3) et une pièce filetée (12) destinée à être ancrée dans l'os.

10. Plaque osseuse (1) selon la revendication 9, **caractérisée en ce que** la pièce de tête (11) a une forme convexe, de préférence sphérique ou conique.

11. Plaque osseuse (1) selon la revendication 9 ou 10, **caractérisée en ce que** la surface d'enveloppe interne (4) et la pièce de tête (5) de la vis de verrouillage de tête (10) présentent des filets mutuellement adaptés.

12. Plaque osseuse (1) selon une des revendication précédentes, **caractérisée en ce que** l'extension périphérique des évidement est d'environ 40°.
